# EUROPEAN PATENT APPLICATION

(11) **EP 1 808 117 A1**
(43) Date of publication of application: **18.07.2007**
(21) Application number: 05795906.6
(22) Date of filing: 24.10.2005
(51) Int. Cl.: A61B 1/00, G02B 23/24

(54) **ENDOSCOPE SHAPE DETECTING DEVICE**

(30) Priority: 26.10.2004 JP 2004311312; 10.11.2004 JP 2004326871
(71) Applicant: Olympus Corporation, Tokyo 151-0072 (JP)
(72) Inventor: ODA, Tomohiko, c/o Olympus Medical Systems Corp., Tokyo 151-0072 (JP); NIWA, Hiroshi c/o Olympus Medical Systems Corp, Tokyo 151-0072 (JP); ONODA, Fumiyuki c/o Olympus Medical Systems Corp, Tokyo 151-0072 (JP); SATO, Minoru c/o Olympus Medical Systems Corp., Tokyo 151-0072 (JP); MIYAKE, Kensuke c/o Olympus Medical Systems Corp., Tokyo 151-0072 (JP); MIYOSHI, Yoshitaka c/o Olympus Medical Systems Corp., Tokyo 151-0072 (JP); AIZAWA, Chieko c/o Olympus Medical Systems Corp., Tokyo 151-0072 (JP)
(74) Representative: von Hellfeld, Axel
(86) International application number: PCT/JP2005/019509
(87) International publication number: WO 2006/046516

(57) **Abstract**

A driving signal is supplied from a transmission block to a magnetic-field generating element arranged on either one of an inside of an endoscope insertion portion and an outside of an endoscope. A magnetic field generated by the magnetic-field generating element is detected by a magnetic-field detecting element arranged on the other and the signal is received by a receiving block. For the signal from the receiving block, control processing including processing for calculating the shape of the endoscope insertion portion is carried out from position information of the magnetic-field generating element or the magnetic-field detecting element arranged inside the endoscope insertion portion. The receiving block provided with an amplification circuit for at least amplifying the signal detected by the magnetic-field detecting element is formed separately from the transmission block and the control block.

## Description

### Technical Field

The present invention relates to an endoscope shape detecting device for detecting and displaying an insertion shape and the like of an endoscope inserted into a body cavity or the like.

### Background Art

Recently, an endoscope has been widely used in the medical field and the industrial field. For example, as a case of inspection of the inside of a lower digestive duct by an operator from an anus side, a skill at a certain level is needed for smooth insertion of an insertion portion into a bent body cavity. Also, during the insertion by the operator, it is necessary to perform smooth insertion by bending operation of a bending portion provided at the insertion portion according to the bending of the digestive duct, and it would be convenient if the location of the tip end position of the insertion portion in the body cavity and the current insertion state of the insertion portion can be known.

Thus, various endoscope shape detection devices for detecting the shape of the insertion state of the endoscope using a magnetic field are proposed, and an endoscope system in which this endoscope shape detecting device is combined with an endoscope device is configured.

For example, Japanese Patent No. 3420084 discloses an endoscope shape detecting device 100 as shown in Figs. 29 and 30.

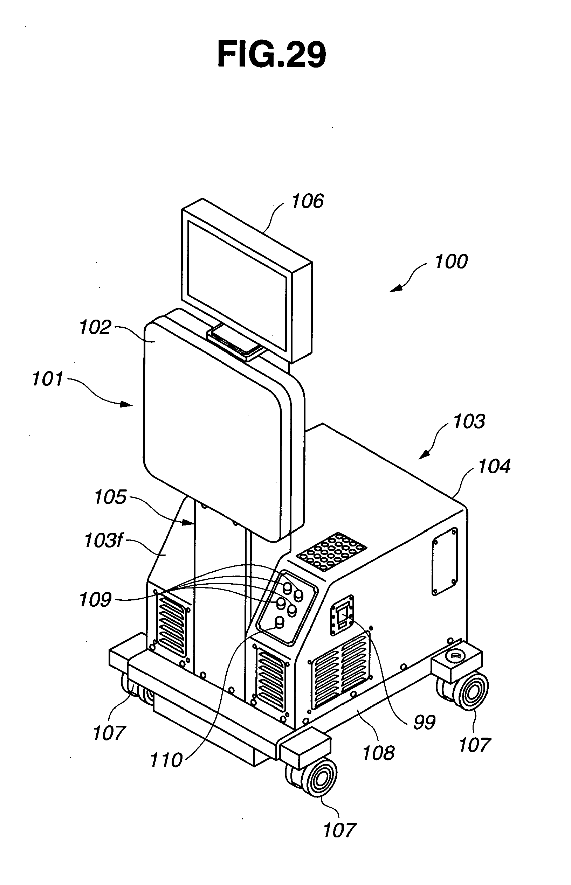

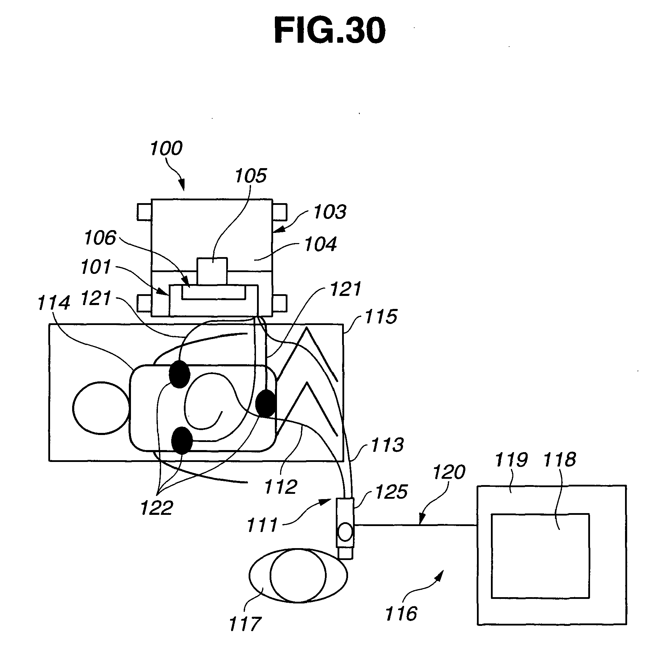

The endoscope shape detecting device 100 shown in Fig. 29 comprises a coil unit 101 in which a detection surface 102 is provided by regularly arranging a plurality of sense coils for detecting a magnetic field generated by a source coil for generating a magnetic field provided at a probe 113 inserted and arranged in an insertion portion 112 of an endoscope 111 shown in Fig. 30, and a main body portion 103.

Inside the main body portion 103, a control device 104 incorporating a transmission block for supplying a driving signal to the source coil provided at the probe 113, a receiving block for receiving a signal transmitted from the sense coil, and a control block provided with a CPU (Central Processing Unit) for executing calculation processing and the like of the endoscope shape on the basis of the received signal detected at the receiving block and the driving signal generated by the transmission block is integrally constructed via a support column 105.

At the support column 105, the coil unit 101 is disposed at the tip end portion of a moving column sliding vertically, and a liquid crystal monitor 106 displaying the insertion shape of the insertion portion 112 is detachably arranged at the upper end. Also, the control device 104 and the support column 105 are fixed onto a base 108 formed by a metal member or the like having rigidity at which a plurality of casters 107 are arranged.

On a front face 103f of the main body portion 103, marker connectors 109 to which marker cables extending from a plurality of markers for indicating a positional relation between the endoscope 111 inserted into the body cavity of a patient and the outside the body cavity during an endoscopic inspection are connected and a probe connector 110 to which a probe 113 having a plurality of source coils for generating a magnetic field arranged is connected, and a power switch 99 for ON/OFF operation of a main power is provided at the side face of the main body portion 103, for example. As shown in Fig. 30, when an endoscopic inspection is to be made, an endoscope device 116 and the endoscope shape detecting device 100 are arranged in the vicinity of an inspection bed 115 on which a patient 114 is loaded so as to configure the endoscope system.

In the vicinity of a position where an operator 117 stands, a cart 119 on which a light source device, a video processor, and an endoscopic observation monitor 118, which are peripheral devices of the endoscope device 116 are loaded is arranged, and the endoscope 111, tubes and cables 120 required for the endoscopic inspection and treatment are connected.

Also, the operator connects the marker cables 121 extending from the plurality of markers 122 to the marker connector 109 (See Fig. 29) of the endoscope shape detecting device 100 arranged adjacent to the inspection bed during the endoscopic inspection and connects the probe 113 on which the plurality of source coils are arranged to the probe connector 110. Also, the operator inserts the probe 113 into the insertion portion 112 through an insertion port of an operation portion 125 of the endoscope 111.

When the insertion portion 112 of the endoscope 111 is inserted into the patient 114, the insertion shape is displayed on the monitor 118. In this conventional example, the coil unit 101 and the control device 104 are integrally configured via the support column 105.

Also, in this conventional example, the transmission block for supplying a driving signal to the source coil, the receiving block for receiving the signal transmitted from the sense coil, and the control block provided with the CPU (Central Processing Unit) for executing the calculation processing and the like of the endoscope shape on the basis of the received signal detected by the receiving block and the driving signal generated at the transmission block are incorporated in the control device 104.

In the above conventional example, since the transmission block, the receiving bock, and the control block are provided in the control device 104, the control signal by the control block and the driving signal by the transmission block, for example, can easily mix in a signal line for inputting a faint signal received (detected) by the sense coil into an amplification circuit of the receiving block and a signal input portion of the amplification circuit as a noise.

If the noise mixes in the faint signal received by the sense coil, that leads to a problem that the S/N of the received signal (detection signal) detected by the sense coil is deteriorated and detection accuracy of the insertion shape is lowered.

The present invention was made in view of the above point and has an object to provide an endoscope shape detecting device which is not easily affected by a noise due to a driving signal and the like and can make shape detection with accuracy.

### Disclosure of Invention

### Means for Solving the Problems

An endoscope shape detecting device of the present invention comprising:
a transmission block for supplying a driving signal to a magnetic-field generating element arranged at one of inside an endoscope insertion portion and outside of an endoscope;
a receiving block for receiving a signal obtained by detecting a magnetic field generated by the magnetic-field generating element by a magnetic-field detecting element arranged on the other; and
a control block for executing control processing including processing for calculating the shape of the endoscope insertion portion from position information of the magnetic-field generating element or the magnetic-field detecting element arranged in the endoscope insertion portion for a signal from the receiving block, wherein
the receiving block provided with an amplification circuit for at least amplifying a signal detected by the magnetic-field detecting element is formed separately from the transmission block and the control block.

By the above configuration, since the signal detected by the magnetic-field detecting element is amplified by the amplification circuit formed separately from the transmission block supplying the driving signal and the control block, even if the driving signal and other signals mixes as a noise, the influence on the amplified signal can be reduced, S/N is not deteriorated, and shape detection can be made with accuracy.

### Brief Description of the Drawings

Fig. 1 is a block diagram illustrating a configuration of an endoscope system provided with a first embodiment of the present invention.
Fig. 2 is a diagram showing an arrangement example of coils incorporated in a coil unit with standard coordinate systems.
Fig. 3 is a block diagram illustrating a configuration of an endoscope device shape detecting device in Fig. 1.
Fig. 4 is a block diagram illustrating internal configurations of a receiving block and a control block in Fig. 3.
Fig. 5 is a block diagram illustrating a more detailed configuration of the receiving block.
Fig. 6 is a timing chart of operations of a 2-port memory and the like.
Fig. 7 is a block diagram illustrating a configuration of the vicinity of a signal transmission portion between the receiving block and the control block in a variation.
Fig. 8 is a plan view illustrating a bed on which a receiving device in a second embodiment of the present invention is provided.
Fig. 9 is a view showing the inside of a bed in a section seen from the side of Fig. 8 in the state where a patient is loaded.
Fig. 10 is a block diagram illustrating a configuration of the vicinity of the signal transmission portion of the receiving block and the control block in a first variation.
Fig. 11 is a block diagram illustrating a configuration of the vicinity of the signal transmission portion of the receiving block and the control block in a second variation.
Fig. 12 is a plan view showing a bed at which a receiving device in a third embodiment of the present invention is provided.
Fig. 13 is a side view of Fig. 12.
Fig. 14 is a side view showing the receiving device in the first variation.
Fig. 15 is a plan view showing a bed at which the receiving device in the second variation is provided.
Fig. 16 is a perspective view illustrating an entire configuration of the endoscope insertion shape detecting device.
Fig. 17A is a perspective view of a sense coil unit seen from the detection surface side.
Fig. 17B is a perspective view of the sense coil unit seen from the back face side.
Fig. 18A is a perspective view showing the sense coil unit and the bed.
Fig. 18B is a perspective view of the sense coil unit mounted to the bed.
Fig. 19 is a perspective view of the sense coil unit mounted to a unit supporting arm.
Fig. 20 is a perspective view of the unit supporting arm mounted to the stand.
Fig. 21 is a perspective view of the unit supporting arm mounted to a side guard frame of an inspection bed.
Fig. 22 is a perspective view of the arm supporting block having a clamp portion.
Fig. 23 is a perspective view showing an entire configuration of the endoscope insertion shape detecting device of another embodiment different from Fig. 16.
Fig. 24 is a perspective view of a state where the sense coil unit is mounted to the unit supporting arm in another embodiment different from Fig. 16.
Fig. 25A is a perspective view of the sense coil unit seen from the back face side in another embodiment different from Fig. 16.
Fig. 25B is a perspective view of the sense coil unit mounted onto the bed.
Fig. 26A is an explanatory view of a state where the unit supporting arm is folded in another embodiment different from Fig. 16.
Fig. 26B is an explanatory view of a state where the unit supporting arm is extended.
Fig. 27A is a perspective view of a state where the sense coil unit is expanded in another embodiment different from Fig. 16.
Fig. 27B is a perspective view of a state where the sense coil unit is folded into halves.
Fig. 28A is a perspective view of a state where the sense coil unit is expanded in another embodiment different from Fig. 16.
Fig. 28B is a perspective view of a state where the sense coil unit is separated at the center.
Fig. 29 is a perspective view showing the endoscope shape detecting device in the conventional example.
Fig. 30 is a view showing a state of an endoscopic inspection by the conventional example.

### Best Mode for Carrying Out the Invention

Embodiments of the present invention will be described referring to the drawings.

### (First embodiment)

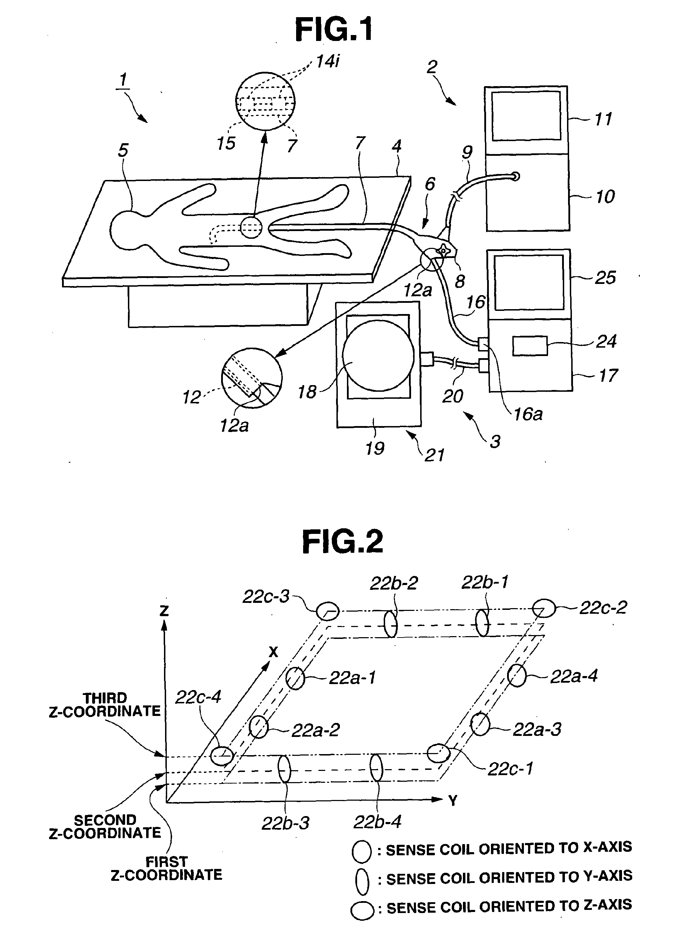

A first embodiment of the present invention will be described referring to Figs. 1 to 7.

As shown in Fig. 1, an endoscope system 1 comprises an endoscope device 2 for performing an endoscopic inspection and an endoscope shape detecting device 3 of the first embodiment used for aiding the endoscopic inspection, and the endoscope shape detecting device 3 is used as insertion aiding means when an endoscopic inspection is made by inserting an insertion portion 7 of an electronic endoscope 6 by an operator into a body cavity of a patient 5 lying on a bed 4.

In the electronic endoscope 6, an operation portion 8 in which a bending operation knob is provided at a rear end of the elongated insertion portion 7 having flexibility is formed, a universal cord 9 is extended from a side portion of the operation portion 8, and the universal cord 9 is connected to a video processor (or a video imaging system) 10 for signal processing and the like.

Into this electronic endoscope 6, a light guide is inserted so that illumination light is transferred from a light source portion within the video processor 10, and the transferred illumination light is emitted from an illumination window provided at the tip end of the insertion portion 7 for illuminating an affected area or the like. An image of a subject such as the illuminated affected area or the like is formed by an objective lens mounted at an observation window provided adjacent to the illumination window by an image pickup device arranged at the image forming position, and the image pickup device makes photoelectric conversion.

The photoelectrically converted signal is given signal processing by a video signal processing portion in the video processor 10 so as to generate a standard video signal and displayed on an image observation monitor 11 connected to the video processor 10.

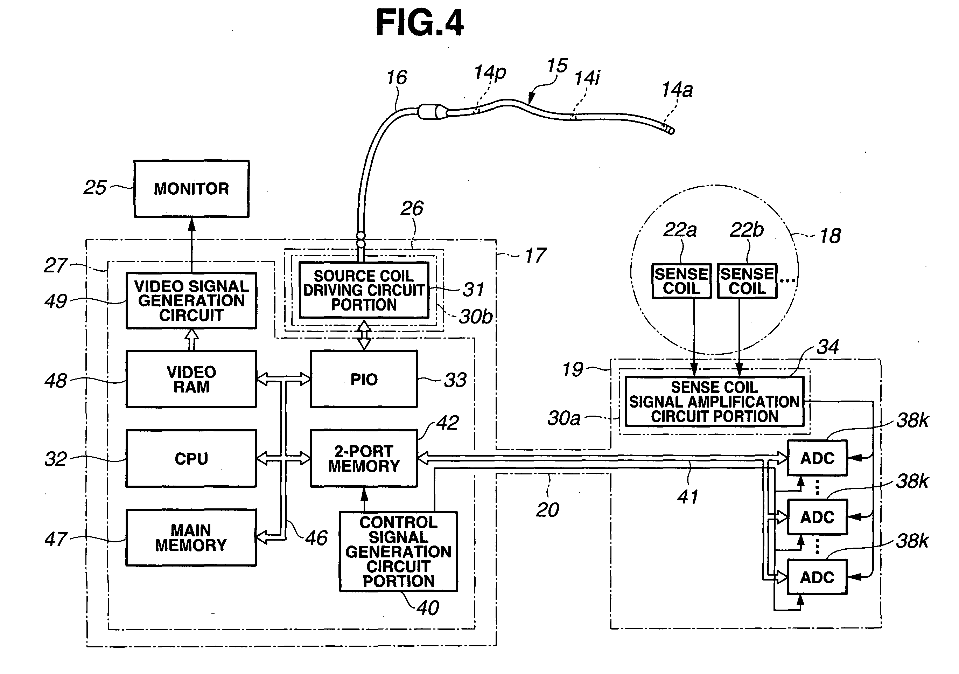

In this electronic endoscope 6, a forceps channel 12 is provided, and source coils 14i are installed in the insertion portion 7 by inserting a probe 15 (See Fig. 4) having sixteen source coils 14a, 14b,... 14p (hereinafter, they are represented by numeral character 14i) as a magnetic-field generating element, for example, from an insertion port 12a of the forceps channel 12.

A source cable 16 extended from the rear end of this probe 15 has a connector 16a at its rear end detachably connected to a signal processing device 17 as a device body of the endoscope shape detecting device 3. And when a driving signal of a given frequency is applied to the source coil 14i as the magnetic-field generating device through the source cable 16 from the signal processing device 17 side as signal transmitting means, the source coil 14i radiates an electromagnetic wave accompanied by the magnetic field to the periphery.

The signal processing device 17 is also connected to a receiving block (or a detecting block) 19 connected to a coil unit 18 arranged in the vicinity of the bed 4 on which the patient 5 lies down through a cable 20. The coil unit 18 and the receiving block 19 form a receiving device (or a detecting device) 21.

To this receiving block 19, the coil unit 18 is mounted capable of movement (elevation) in the vertical direction, and a plurality of sense coils as magnetic-field detecting devices are arranged in this coil unit 18.

Describing more specifically, there are arranged twelve sense coils, for example (hereinafter, they are represented by reference character 22j): sense coils 22a-1, 22a-2, 22a-3, and 22a-4 oriented to the X-axis, for example, whose central Z-coordinate is a first Z-coordinate, sense coils 22b-1, 22b-2, 22b-3, and 22b-4 oriented to the Y-axis whose central Z-coordinate is a second Z-coordinate different from the first Z-coordinate, and sense coils 22c-1, 22c-2, 22c-3, and 22c-4 oriented to the Z-axis, for example, whose central Z-coordinate is a third Z-coordinate different from the first and the second Z-coordinates.

The sense coil 22j is connected to the receiving block 19 through a cable, not shown, extended from the coil unit 18. This receiving block 19 amplifies or the like a signal detected by the sense coil 22j, as will be described later, and converts the analog signal to a digital signal by A/D conversion. And the digital signal is transmitted to the signal processing device 17 through a signal cable 20.

And in a control block 27 in the signal processing device 17, calculation processing of position of each source coil 14i, estimation processing of the insertion portion shape by calculation of each position, display processing for displaying an image of the estimated insertion portion shape and the like are carried out, and the image of the insertion portion shape is displayed on a liquid crystal monitor 25.

In the present embodiment, the receiving block 19 for amplifying a signal received by the sense coil 22j detecting a faint signal generated by the source coil 14i in this way or the like and converting it to a signal which is not easily affected by a noise, a transmission block 26 for generating a driving signal for driving the source coil 14i, and the signal processing device 17 including the control block 27 for control processing including calculation of the insertion shape are made separate so that they can be arranged away form each other.

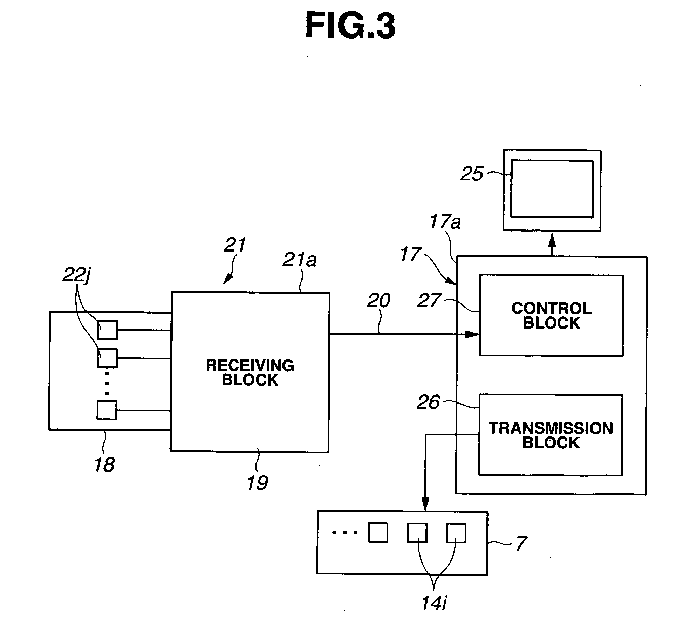

Specifically, as shown in Fig. 3, in the present embodiment, the endoscope shape detecting device 3 is configured so that the signal processing device 17 incorporating the transmission block 26 and the control block 27 is connected to the receiving device 21, separate from the signal processing device 17, by the signal cable 20 for signal transmission. That is, the signal processing device 17 and the receiving device 21 are stored in separated cases 17a and 21 a so that the distance between the both can be increased or they can be arranged separately from each other. And influence of an electric signal on the both as a noise can be reduced.

Also, the receiving device 21 comprises the coil unit 18 incorporating the plurality of sense coils 22j and the receiving block 19, and the receiving block 19 is configured to amplify a signal detected by the sense coil 22j and after processing such as AD (analog/digital) conversion or the like, to transmit it to the control block 27 of the signal processing device 17 by the signal cable 20.

The control block 27 detects a position of each source coil 14i from the AD converted signal and then, carries out processing to display the shape of the insertion portion 7 and transmits a video signal of the endoscope (insertion) shape to the liquid crystal monitor 25 as display means of endoscope shape so that the endoscope shape is displayed on the display surface of the liquid crystal monitor 25. Also, the control block 27 carries out control processing of supply timing and the like of the driving signal of the transmission block 26.

The signal processing device 17 is provided with an operation panel 24 (See Fig. 1) for operating the device by a user.

As shown in Fig. 4, in the probe 15 installed in the insertion portion 7 of the electronic endoscope 6, sixteen source coils 14i for generating a magnetic field are arranged with a predetermined interval as mentioned above, and the source coils 14i are connected to a source coil driving circuit portion 31 for generating driving signals with sixteen frequencies different from each other, for example, constituting the transmission block 26.

The source coil driving circuit portion 31 drives each source coil 14i with a sinusoidal-wave driving signal with a frequency different from each other, for example, and the respective driving frequency is set by driving-frequency setting data (also noted as driving frequency data) stored in driving-frequency setting data storing means or in driving-frequency setting data memory means, not shown, within the source coil driving circuit portion 31.

The driving-frequency data is stored in the driving-frequency data storing means (not shown) within the source coil driving circuit portion 31 through a PIO (parallel input/output circuit) 33 by a CPU (central processing unit) for calculation processing and the like of the endoscope shape in the control block 27.

On the other hand, the sense coil 22j in the coil unit 18 is connected to a sense-coil signal amplification circuit portion 34 constituting the receiving block 19.

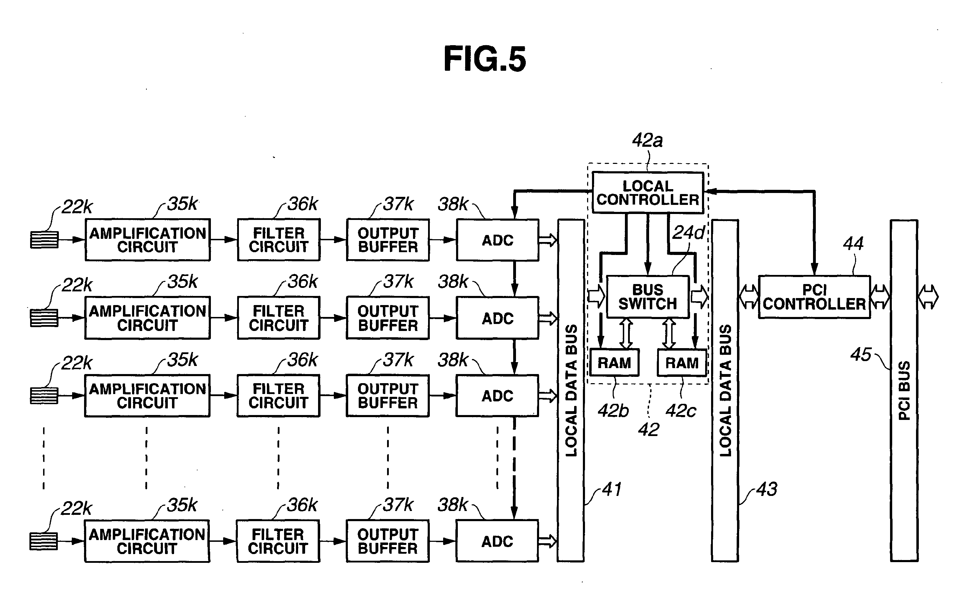

In the sense-coil signal amplification circuit portion 34, twelve single-core coils 22k constituting the sense coil 22j as shown in Fig. 5 are connected to amplification circuits 35k, respectively, so as to provide twelve processing systems, and a faint signal detected by each single-core coil 22k is amplified by the amplification circuit 35k, has a band through which plural frequencies generated by the source coil group at a filter circuit 36 pass and is outputted to an output buffer 37k after having an unnecessary component removed. Then, the signal is converted by an ADC (analog/digital converter) 38k to a digital signal readable by the control block 27.

The receiving block 19 is comprised by the sense-coil signal amplification circuit portion 34 and the ADC 38k, and the sense-coil signal amplification circuit portion 34 is comprised by the amplification circuit 35k, the filter circuit 36k, and the output buffer 37k.

Also, in the present embodiment, in order to reduce the influence by noise hard to affect, in the receiving block 19 in Fig. 4, the sense-coil signal amplification circuit portion 34 is stored in a shield case 30a having a function to electromagnetically shield the noise and the like as shown by a two-dot chain line, for example.

Also, the entire receiving block 19 may be stored in a shield case. In the present embodiment, since a clock is also applied to the ADC 38k in the receiving block 19, in order to prevent the clock from mixing as a noise in the sense-coil signal amplification circuit portion 34 handling a faint signal, only the sense-coil signal amplification circuit portion 34 is shielded. When the sense-coil signal amplification circuit portion 34 is to be shielded, only the amplification circuit 35k portion shown in Fig. 5 may be shielded.

Similarly, the transmission block 26 is stored in a shield case 30b shown by a two-dot chain line in Fig. 4 in order to reduce a noise radiated to the outside.

Also, as shown in Fig. 4, an output from the sense-coil signal amplification circuit portion 34 is transmitted to the ADC 38k and converted to digital data with a predetermined sampling frequency by a clock supplied from a control signal generation circuit portion 40. Also, the digital data generated by the ADC 38k is transmitted through a data transmission line 41 formed by a plurality of signal lines inserted through the signal cable 20 by the control signal from the control signal generation circuit portion 40 and written in a 2-port memory 42 to which the transmission line 41 is connected.

Also, a clock for AD conversion and a control signal (specifically, AD conversion start signal, too) from the control signal generation circuit portion 40 are also transmitted to the ADC 38k through the signal line in the signal cable 20.

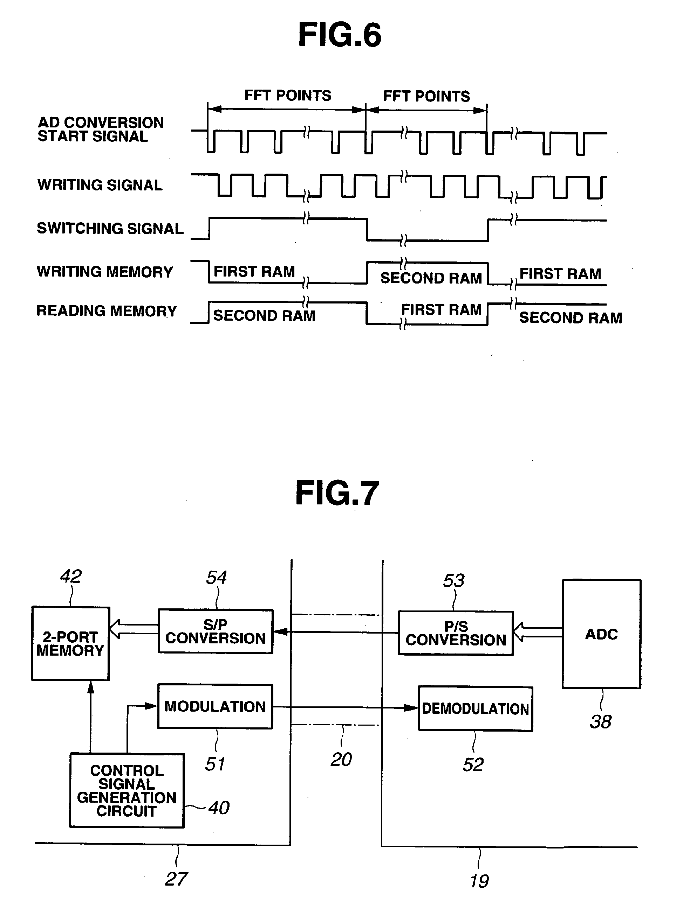

The 2-port memory 42 functionally comprises, as shown in Fig. 5, a local controller 42a, a first RAM 42b, a second RAM 42c, and a bus switch 42b. And by timing as shown in Fig. 6, the ADC 38k starts A/D conversion by the AD conversion start signal from the local controller 42a. And while the bus switch 42d switches the RAM 42b, 42c by a switching signal from the local controller 42a, it takes in data all the time after power on using the first RAM 42b, 42c as a reading memory and a writing memory alternately.

Returning to Fig. 4, again, the CPU 32 reads out the digital data written in the 2-port memory 42 by the control signal from the control signal generation circuit portion 40 through an internal bus 46 comprised by a local data bus 43, a PCI controller 44, and a PCI bus 45 (See Fig. 5).

The CPU 32 carries out frequency extraction processing (fast Fourier transformation: FFT) or synchronous detection for the digital data using the main memory 47 as will be described later so as to separate/extract to magnetic-field detection information of a frequency component corresponding to a driving frequency of each source coil 14i. The CPU 32 calculates a space position coordinate of each source coil 14i provided in the insertion portion 7 of the electronic endoscope 6 from each digital data of separated magnetic-field detection information.

Also, from the calculated position coordinate data, the insertion state of the insertion portion 7 of the electronic endoscope 6 is estimated, and display data forming an endoscope shape image is generated and outputted to the video RAM 48. The data written in the video RAM 48 is read out by a video signal generation circuit 49, converted to an analog video signal and outputted to the liquid crystal monitor 25.

The liquid crystal monitor 25 displays the insertion shape of the insertion portion 7 of the electronic endoscope 6 on the display screen upon input of the analog video signal.

At the CPU 32, the magnetic-field detection information corresponding to each source coil 14i, that is, an electromotive force (amplitude value of a sinusoidal signal) generated at the single-core coil 22k constituting each sense coil 22j and phase information are calculated. The phase information includes a polarity ± of the electromotive force.

Action of the present embodiment configured as above will be described.

As shown in Fig. 1, the probe 15 is inserted into the forceps channel 12 of the electronic endoscope 6, this electronic endoscope 6 is connected to the video processor 10, and the source cable 16 of the probe 15 is connected to the signal processing device 17.

Also, the operator places the receiving block 19 to which the coil unit 18 is mounted in the vicinity of the patient 5, and the receiving block 19 is connected to the signal processing device 17 by the signal cable 20.

And the operator inserts the insertion portion 7 of the electronic endoscope 6 into the body cavity of the patient 5. Each source coil 14i built in the probe 15 installed in the insertion portion 7 generates a magnetic field around by the driving signal from the transmission block 26.

The generated magnetic field is detected by each sense coil 22j of the coil unit 18 and then, amplified by the amplification circuit 35k, respectively. The amplified signal goes through the filter circuit 36k and the like and is converted to a digital signal (digital data) by the ADC 38k. Each digital data is stored in the 2-port memory 42 constituting the control block 27 of the signal processing device 17 through the signal cable 20.

The digital data stored in the 2-port memory 42 is given fast Fourier transformation, synchronous detection or the like and a signal component of the frequency of the driving signal is separated/extracted by the CPU 32 constituting the control block 27. And the CPU 32 further calculates a spatial position coordinate of each source coil 14i provided in the insertion portion 7 of the electronic endoscope 6 form each separated/extracted digital data, estimates the insertion state of the insertion portion 7 of the electronic endoscope 6 from the calculated position coordinate data, and generates display data for forming an endoscope shape image and outputs it to the video RAM 48.

The data written in the video RAM 48 is read out by the video signal generation circuit 49, converted to an analog video signal and outputted to the liquid crystal monitor 25. The liquid crystal monitor 25 displays the insertion shape of the insertion portion 7 of the electronic endoscope 6, that is, the endoscope insertion shape on the display screen, upon input of the analog video signal.

Then, the operator can estimate the shape of the insertion portion 7 in the body cavity and the position of the vicinity of the tip end portion by referring to the displayed endoscope insertion shape and can utilize it for aiding smooth insertion.

In the present embodiment, the faint detection signal detected by the sense coil 22j is amplified by the amplification circuit 35k in the receiving block 19 provided separately from the signal processing device 17 incorporating the transmission block 26 and the control block 27, and further AD-converted to a digital signal and then, transmitted to the control block 27 by the signal cable 20 for processing of endoscope shape display.

That is, in the present embodiment, the faint detection signal detected by the sense coil 22j is given amplification and AD conversion processing while being hardly affected by the driving signal of the transmission block 26 or a clock signal in the control block 27 with operation of the CPU 32.

Also, the digital signal after AD conversion is hardly affected by a noise as compared with the detection signal detected by the sense coil 22j.

Therefore, according to the present embodiment, the endoscope shape display processing is carried out from signal detection with good S/N, and endoscope shape display with accuracy is enabled.

Also, according to the present embodiment, by providing the signal processing device 17 and the receiving block 19 separately, they can be arranged at appropriate positions or in small spaces, respectively. For example, since the receiving block 19 portion can be made compact, it can be arranged closer to the patient 5 so that the magnetic field generated by the source coil 14i can be detected at a closer position, that is, in the state with good S/N. On the other hand, if it is integrated as in the conventional example, a large space is needed at installation, which leads to a problem of restriction on an installation place or the like.

According to the present embodiment, by providing the receiving block which can be driven by a relatively small power supply separately form the signal processing device 17 driven by a large current such as the transmission block 26 and the control block 27, the noise which easily mixes through a power supply circuit can be also reduced more easily.

Fig. 7 shows configuration of the vicinity of a signal transmission portion between the control block 27 and the receiving block 19 in a variation. In the present variation, when a clock and a control signals are to be sent from the control signal generation circuit portion 40 of the control block 27, it is modulated by a modulation circuit 51 and transmitted to the receiving block 19 by a single signal line (except a ground line), for example, while it is demodulated by a demodulation circuit 52 at the receiving block 19 so as to regenerate the clock and the control signals and supply them to an ADC portion 38 (comprised by twelve ADC 38k).

On the other hand, the digital data generated by the ADC portion 38 is converted to serial data by a parallel/serial conversion circuit (abbreviated as P/S conversion in Fig. 7) 53 and transmitted to a serial/parallel conversion circuit (abbreviated as S/P conversion in Fig. 7) 54 of the control block 27 at a high speed by a serial data transmission line.

At the serial/parallel conversion circuit 54, the data is converted to parallel data, and the parallel data is written in the 2-port memory 42.

The other configurations are the same as those of the first embodiment. According to the present variation, signals can be transmitted/received by the smaller number of signal cables 20. The similar effects as those of the first embodiment can be also obtained.

### (Second embodiment)

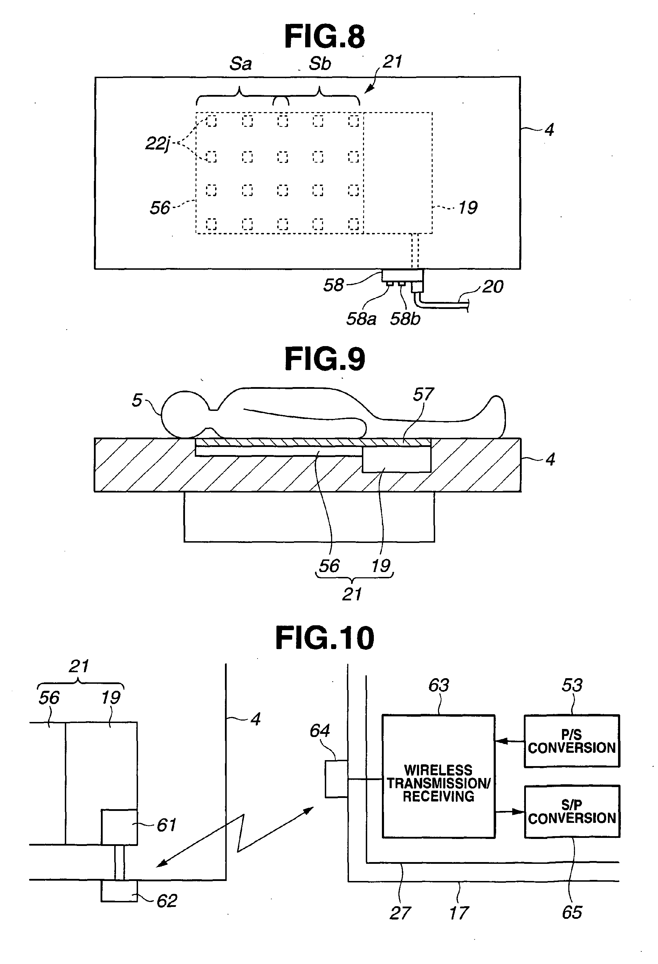

Next, a second embodiment of the present invention will be described referring to Figs. 8 and 9. In the present embodiment, the receiving device 21 is provided at the bed 4. Fig. 8 shows a plan view of the bed 4, and Fig. 9 shows a sectional structure when seen from the side face side in the state where the patient 5 is loaded.

As shown in Fig. 9, a recess portion is formed on an upper face to be the vicinity of the substantial center in the bed 4 where the patient 5 is to be loaded, in which a coil unit 56 formed in a flat plate state is stored, and in a recess portion adjacent to it, the receiving block 19 to which the sense coils 22j of the coil unit 56 are connected is also stored.

The upper faces of the coil unit 56 and the receiving block 19 are covered by a cover 57, and the upper face of the cover 57 is made flush with the upper face of the bed 4.

Also, as shown in Fig. 8, a connector portion 58 connected to the receiving block 19 by a cable is provided on the side face of the bed 4, and to the connector portion 58, a connector at one end of the signal cable 20 is detachably connected, and the other end of the signal cable 20 is connected to the control block 27 of the signal processing device 17.

Also, in the present embodiment, more sense coils 22j than in the first embodiment are arranged in the coil unit 56, and the sense coil 22j to be actually used can be selected by selection switches 58a, 58b provided at the connector portion 58, for example.

For an inspection of an upper part of the patient 5 such as an upper digestive duct, for example, twelve sense coils 22j of an upper part unit portion Sa arranged at a position to be the upper part side of the patient 5 to be loaded are used, while for an inspection of a lower digestive duct, the sense coils 22j of a lower part unit portion Sb arranged at the lower part are used so that the position of each source coil 14i can be detected in the state with good S/N.

According to the present embodiment, since the magnetic field can be detected in the state extremely close to the patient 5, the amplitude of the detection signal detected by the sense coil 22j can be made large, and endoscope shape detection with good S/N and endoscope shape display with accuracy are realized.

Also, a labor to arrange the receiving device 21 around the bed 4 is not needed any more. Also, since the number of devices to be arranged around the operator is reduced, the operator can perform operation more easily. The other similar working effects as those of the first embodiment can be also obtained.

Fig. 10 shows configuration of the vicinity of a signal transmission portion of the receiving block 19 and the control block 27 in the first variation. In the present variation, in the receiving device 21 shown in Fig. 8, a wireless transmission/receiving circuit 61 for signal processing for wireless transmission/receiving is provided in the receiving block 19, for example, and transmission/receiving of a signal is configured to be made in a wireless (electric wave) manner by an antenna 62 which can be installed upright on the side portion of the bed 4.

Also, a wireless transmission/receiving circuit 63 is provided on the control block 27 side of the signal processing device 17, and an antenna 64 connected to the wireless transmission/receiving circuit 63 is provided. More specifically, the P/S conversion circuit 53 shown in Fig. 7 and an S/P conversion circuit 65 provided instead of the demodulation circuit 52 are connected to the wireless transmission/receiving circuit 63, for example.

By configuring the receiving block 19 side substantially similarly, transmission/receiving of signals is made capable by the both antennas 62, 64. According to the present variation, since there is no need to connect the receiving block 19 and the control block 27 (signal processing device 17) by the signal cable 20, favorable operability can be ensured. The other similar effects as those of the second embodiment can be also obtained,

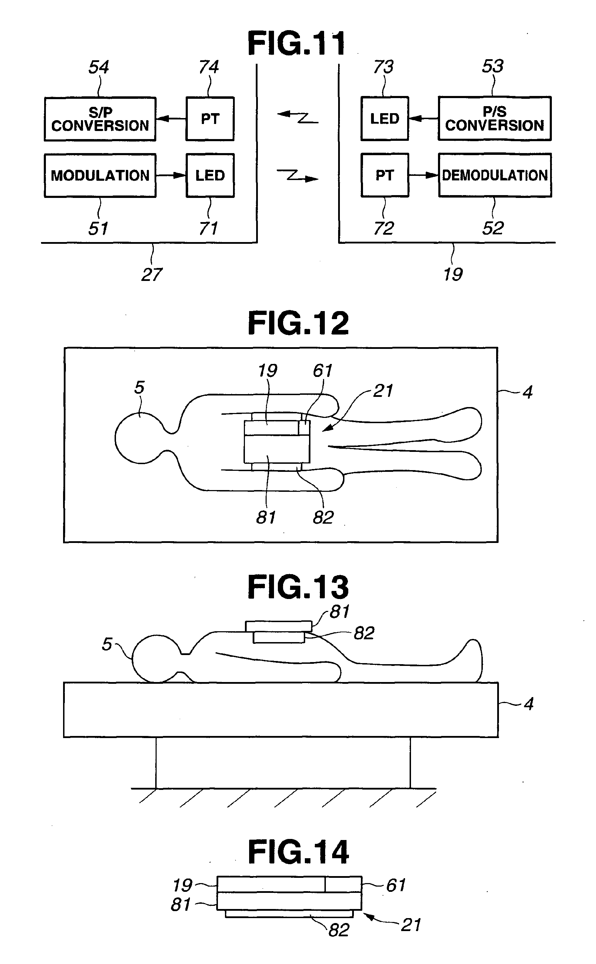

Fig. 11 shows configuration of the vicinity of a signal transmission portion of the receiving block 19 and the control block 27 in a second variation. In the present variation, a light emitting diode (abbreviated as LED) 71 as a light emitting element, for example, is made to emit light by an output signal of the modulation circuit 51 in Fig. 7, for example. Also, at an input end of the demodulation circuit 52, a photo transistor (abbreviated as PT) 72 as a light receiving element, for example, is connected so that the light of the LED 71 is received by this PT 72.

Also, the output signal of the P/S conversion circuit 53 is configured to emit light by the LED 73 and this light is received by the PT 74 and inputted to the S/P conversion circuit 54.

The present variation also has substantially the same working effects as those of the first variation. Though not shown, as a third variation, such configuration is possible that transmission between the LED 71 and the PT 72 as well as the LED 73 and the PT 74 in the second variation is made by an optical fiber. In this case, the present invention can be applied to a case where the LED 71 and the PT 72 as well as the LED 73 and the PT 74 are not opposed to each other.

### (Third embodiment)

Next, a third embodiment of the present invention will be described referring to Figs. 12 and 13. In the present embodiment, the receiving device 21 is affixed to the patient 5 for use. Fig. 12 is a plan view seen from above, and Fig. 13 is a side view seen from the side.

As shown in Fig. 12, in the receiving device 12 in the present embodiment, a coil unit 81 and the receiving block 19 are arranged adjacently to each other substantially in the flat plate state. Also, to the receiving block 19, the wireless transmission/receiving circuit 61 (including a small-sized antenna) is connected.

On the bottom surface side of the receiving device 21, an adhesive tape 82 to be detachably attached to the surface of an abdomen or the like of the patient 5 to be inspected by affixing, for example, is provided. The detachably attaching means is not limited to the adhesive tape 82, but a belt-state one may be used, for example.

In Fig. 12 or 13, the coil unit 81 and the receiving block 19 are provided adjacently in the horizontal direction so as to form a flat plate, but as in a first variation shown in Fig. 14, they may be stacked in the thickness direction. The signal processing device 17 in the present embodiment is configured to be provided with the wireless transmission/receiving circuit 63 as shown in Fig. 10, for example.

The working effects of the present embodiment and the first variation are similar to those of Fig. 10, but since the receiving device 21 is attached to the patient 5, there is a merit that even after the body position of the patient 5 is changed, the endoscope shape can be displayed substantially in the same state as before the change.

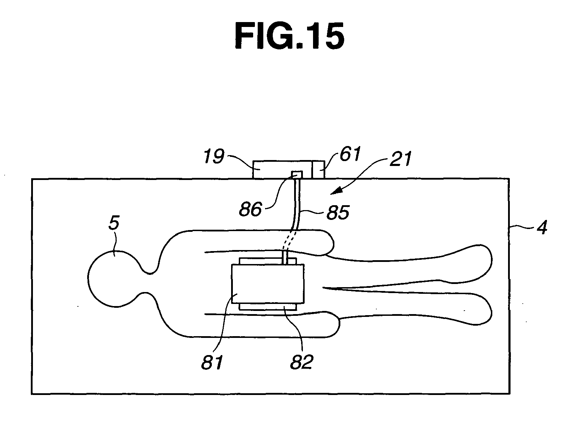

Fig. 15 shows the receiving device 21 in a second variation. In the present variation, the coil unit 81 attached by an adhesive, for example, to the patient 5 in the third embodiment and the receiving block 19 (including the antenna 62) are detachably connected to each other through a flexible print circuit board 85, for example.

A connector 86 provided at the end of the flexible print circuit board 85 is detachably connected to a connector receiver provided at the receiving block 19.

According to the present variation, since only the coil unit 81 used for detection of the endoscope shape is attached to the patient 5, a sense of discomfort given to the patient 5 can be reduced. The other working effects are substantially the same as those of the third embodiment.

Also, in the present variation, by making the coil unit 81 detachable, this portion can be produced at a lower cost and can be also made disposable. Only the flexible print circuit board 85 for detachably connecting the both may be made disposable.

In each of the above embodiments, the receiving block 19 is configured to be provided with the amplification circuit 35k for amplifying the signal detected by the sense coil 22j and the ADC 38k, but not limited to this, the signal amplified by the amplification circuit 35k may be transmitted to the control block 27 side by the signal cable 20 to be given AD conversion on the control block 27 side. In this case, too, since the faint signal is amplified, an influence of the noise can be reduced, and shape detection in the state with good S/N is enabled.

When the driving signal to be supplied to the source coil 14i from the transmission block 26 mixes in the receiving block 19 as a noise, since it has the same frequency as that of the signal component by the magnetic-field generation by the source coil 14i to be detected, its influence is large. Thus, the case where the receiving block 19 is made separate at least from the transmission block 26 also belongs to the present invention.

In the above embodiments, the configuration is described that the source coil 14i for generating a magnetic field is arranged in the insertion portion 7 of the electronic endoscope 6 and the sense coil 22j is arranged outside the body, but the present invention is not limited to this but it may be so configured that the sense coil 22j is arranged in the insertion portion 7 and the source coil 14i is arranged outside the body.

This case is also configured such that the detection signal detected by the sense coil 22j is detected by the receiving block 19. When this is applied to the first embodiment, for example, a connector 16a of the source cable 16 is connected to the receiving block 19. However, the sense coil 22j is arranged in the probe 15 of the source cable 16.

According to each of the above embodiments, since the signal detected by a magnetic-field detecting element is at least amplified at a separate portion, deterioration of S/N over the noise due to the driving signal and the like is prevented and shape detection with accuracy is enabled.

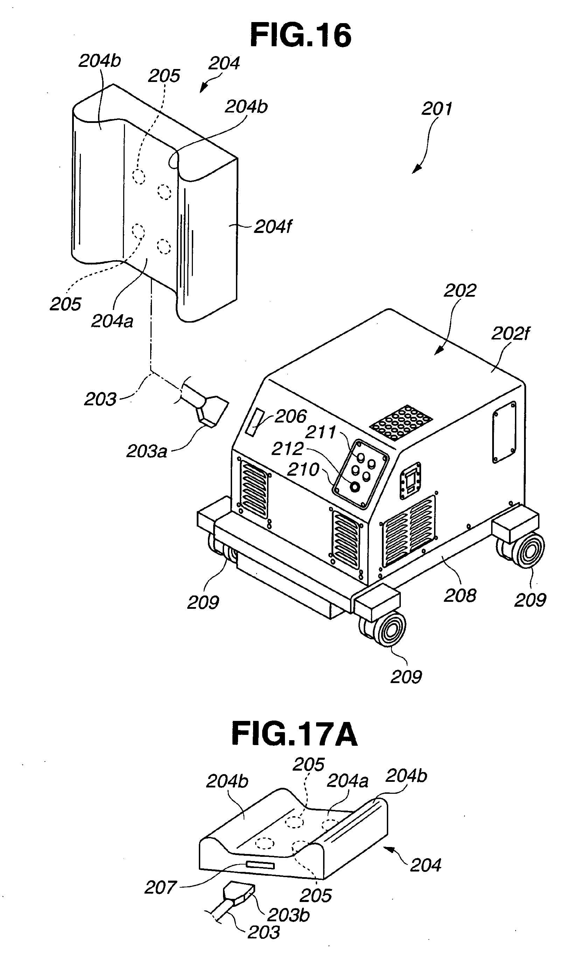

Next, an endoscope insertion shape detecting device which can easily set an external unit arranged outside the endoscope insertion portion, more specifically, on the periphery of the patient at an optimal position and moreover at a position independent of the patient, which is easy to be used, will be described referring to Figs. 16 to 22. First, the background of this endoscope insertion shape detecting device will be described below.

In general, endoscopes are widely employed not only for medical use but also for industrial use. For example, a medical endoscope is used for carrying out observation of an inspected portion or required treatment through a treatment instrument insertion channel by inserting an elongated insertion portion with flexibility into a body cavity.

Inside the body cavity represented by a colon and a small intestine is not in the straight shape but has a bent shape, and it is difficult for an operator to grasp where the tip end portion of the endoscope insertion portion inserted into the body cavity is located or in what shape the endoscope insertion portion is bent, and experience and skill are needed for smooth endoscopic inspections.

In order to handle this situation, a technology that an X-ray is irradiated from outside and the endoscopic inspection is carried out while the insertion state such as the insertion position and the insertion shape of the endoscope insertion portion inserted into the body cavity is checked with an X-ray monitor is proposed. However, since X-ray irradiation spots are limited, accurate detection of the insertion state of the endoscope insertion portion is difficult.

Japanese Patent No. 3283478 and Japanese Patent No. 3389518 disclose endoscope insertion shape detecting devices which can easily detect the insertion state of the endoscope insertion portion from outside using a magnetic field.

In these patents, a source coil is provided at the endoscope insertion portion and a sense coil unit is disposed outside the patient. And a magnetic field generated by the source coil is detected by a plurality of sense coils incorporated in the sense coil unit and a detected image is displayed on a monitor so that the insertion shape of the endoscope insertion portion inserted into the body cavity can be easily grasped from outside.

However, in the art disclosed in the former patent, since the sense coil unit is integrated into the device body through a support column, movement is allowed only in a range supported by the support column, and not only that placement at an optimal position is difficult for detection of the insertion state of the endoscope insertion portion but since the device body is disposed in the vicinity of the sense coil unit all the time, it easily obstructs an endoscopic inspection and becomes inconvenient in use.

On the other hand, in the art disclosed in the latter patent, since the sense coil unit is made in a sheet state to be wrapped around the patient for use and connected to the device body through a cable, the device body can be disposed at a position not obstructing an endoscopic inspection. Also, since the sense coil unit is wrapped around the patient for use, the insertion state of the endoscope insertion portion can be detected at an optical position.

However, in the art disclosed in the latter patent, the sheet-state sense coil unit should be attached/detached with respect to the patient at every inspection or treatment, which makes attachment/detachment work cumbersome. Also, if the position of the sense coil unit is displaced at change of the body position of the patient during the endoscopic inspection, the endoscopic inspection should be stopped and the sense coil unit should be attached again, which makes the device inconvenient in use.

Then, the present invention has an object to provide an endoscope insertion shape detecting device which can easily set an external unit at an optical position and can be arranged independently of a patient, which is convenient to be used.

In order to achieve this object, in an endoscope insertion shape detecting device including a plurality of magnetic-field generating elements, driving signal generating means for generating a magnetic field by supplying a driving signal to the plurality of magnetic-field generating elements, magnetic-field detecting element for detecting the magnetic field generated by the plurality of magnetic-field generating elements, either one of the magnetic-field generating element and the magnetic-field detecting element being disposed in an endoscope insertion portion with the other of the magnetic-field generating element and the magnetic-field detecting element disposed on an external unit outside the endoscope insertion portion, and a device body being provided having a calculation control portion so that a signal detected by the magnetic-field detecting element is received for calculating the shape of the endoscope insertion portion, wherein
the external unit is separated from the device body and disposed at a position independent of the patient. And the above object is achieved.

An embodiment (hereinafter referred to as a fourth embodiment for convenience) of the endoscope insertion shape detecting device will be described below referring to Figs. 16 to 22.

As shown in Fig. 16, an endoscope insertion shape detecting device 201 comprises a device body 20 and a sense coil unit 204, which is an example of an external unit (coil unit) to be connected to the device body 202 through a cable 203.

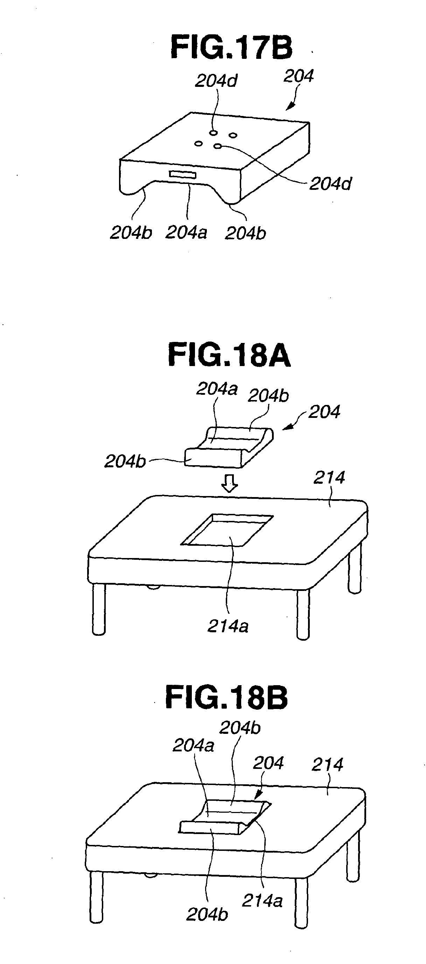

As shown in Figs. 17A and 17B, a detecting surface 204a of the sense coil unit 204 is formed as substantially flat, and a plurality of sense coils 205 are incorporated on the detecting surface 204a side as magnetic-field detecting elements. Moreover, a support portion 204b is formed in the projecting state on both sides of the detecting surface 204a of the sense coil unit 204, while a mounting screw hole 204d is screwed on the back face, and a fixing member for fixing (mounting) the sense coil unit 204 is formed.

Also, connector receivers 206, 207 are provided at the device body 202 and the sense coil unit 204, and to each of the connector receivers 206, 207, connectors 203a, 203b provided at both ends of the cable 203 are connected.

Each sense coil 205 detects a magnetic field generated by a source coil disposed at an insertion portion of the endoscope, not shown, and transmits the detection signal to the device body 202 side through the cable 203. Also, power is supplied to each sense coil 205 from the device body 202 through the cable 203. Since the configuration of the magnetic-field generating element (source coil) disposed at the insertion portion of the endoscope is known as disclosed in the above-mentioned Japanese Patent No. 3389518, the description here is omitted.

Also, a base 208 is provided at the bottom portion of the device body 202, and casters 209 are fixed at the base 208 to enable free movement. In the device body 202, there are provided driving signal generating means for generating a magnetic field by supplying a driving signal to a plurality of magnetic-field generating elements disposed at the insertion portion of the endoscope, a calculation control portion mainly including a computer such as a microcomputer for receiving a signal detected by each sense coil 205 and carrying out calculation processing and the like of the insertion shape of the endoscope insertion portion, and an image processing calculation portion for executing image processing of the insertion shape of the endoscope insertion portion calculated by the calculation control portion and displaying it on a monitor, not shown.

Also, an operation portion 210 is disposed at the front surface of the device body 202. In this operation portion 210, marker connectors 211 for connecting marker cables extending from a plurality of markers indicating the position relation between the endoscope inserted into the body cavity of the patient and the outside of the body cavity and a power switch 212 for power ON/OFF and the like are provided.

The sense coil unit 204 is connected to the device body 202 through the flexible cable 203. That is, as shown in Fig. 16, a case 20f of the sense coil unit 204 and a case 202f of the device body 202 are separated from each other and connected by the cable 203 for transmitting a signal.

Therefore, the sense coil unit 204 is not bound by the position where the device body 202 is installed, but the mounting position can be freely set. A state where the sense coil unit 204 is mounted will be described below based on Figs. 18A to 22.

Fig. 18B shows a state where the sense coil unit 204 is fixed to an inspection bed (abbreviated as a bed) 214. Fig. 18A shows a state before the sense coil unit 204 is mounted to the bed 214.

On the bed 214, a recess portion 214a as a positioning portion for storing the sense coil unit 204 is formed. The depth of this recess portion 214a is set so that when the sense coil unit 204a is attached to the recess portion 214a, the detecting surface 204a becomes substantially flush with the surface of the bed 214. Therefore, in the state where the sense coil unit 204 is mounted to the bed 214, the sense coil unit 204 does not obstruct the patient. That is, the sense coil unit can be mounted at a position independently of the patient.

However, in the present embodiment, two support portions 204b in the projecting state are provided at the sense coil unit 204 as shown in Fig. 18 and the like and the abdominal part of the patient lying on the bed 214 is positioned so that the patient is supported in a posture or a position that the insertion shape can be easily detected by a detection signal of the sense coil unit 204.

In the present embodiment constructed as above, the patient is made to lie on the inspection bed 214, and the sense coil unit 204 is opposed to a portion corresponding to a position where the endoscope insertion portion is to be inserted such as an abdominal part. At that time, since the patient is supported on the sense coil unit 204 by the support portions 204b formed at both sides of the sense coil unit 204, the insertion state of the endoscope insertion portion can be detected at an optimal position. In this case, since the device body 202 can be retreated and placed at a position not obstructing the endoscopic inspection, usability is good.

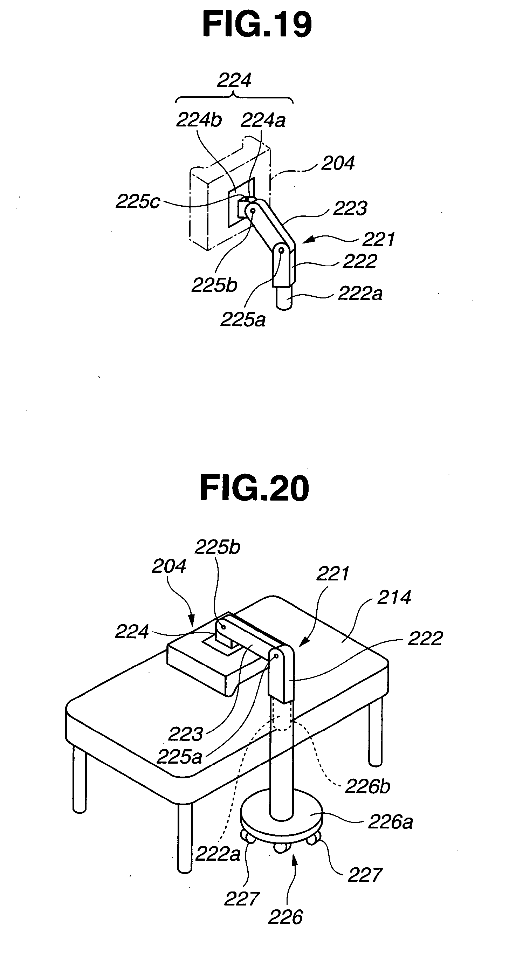

Figs. 19 to 21 show a state where the sense coil unit 204 is fixed to a support arm (unit support arm) 221 as a unit support member so that the sense coil unit 204 can be fixed at an arbitrary position through the unit support arm 221.

As shown in Fig. 19, the unit support arm 221 has a stand mounting portion 222 provided at its lower end. A boss shaft 222a is projected at the lower end of the stand mounting portion 222. Also, at the upper end, a base end of an arm portion 223 as holding means is supported by the boss shaft 222 rotatably in the vertical direction in the figure through a shaft 225a.

Also, a support portion 224a provided at the unit mounting portion 224 is supported at the upper end of the arm portion 223 rotatably in the vertical direction in the figure through a shaft 225b. Moreover, at the tip end of this support portion 224a, a unit mounting surface 224b is supported through a shaft 225c rotatably in the horizontal direction in the figure.

To this unit mounting surface 224b, the back face of the sense coil unit 204 is fixed. On the back face of the sense coil unit 204, the mounting screw hole 204d is screwed for installation, and by screwing a screw into this mounting screw hole 204d via a screw through hole (not shown) drilled in the unit mounting surface 224b, the sense coil unit 204 is fixed to the unit mounting portion 224. In this case, since the cable 203 connected to the sense coil unit 204 is disposed through a space formed inside the unit support arm 221, wiring can be made compact.

Fig. 20 shows an example where the unit support arm 221 to which the sense coil unit 204 is mounted is attached to a stand 26 as a receiving member. The stand 226 has casters 227 fixed to a base 226a formed at the lower end to enable free movement. Also, at the upper end of the stand 226, a recess portion 226b for supporting the boss shaft 222a projected at the lower end of the unit support arm 221 is formed.

In this construction, by attaching the boss shaft 222a projected at the lower end of the unit support arm 221 to the recess portion 226b formed at the stand 226, the sense coil unit 204 can be moved and set at an arbitrary position and direction in a three-dimensional space by rotating the stand 221 in the three-dimensional direction without moving the patient lying on the bed 214.

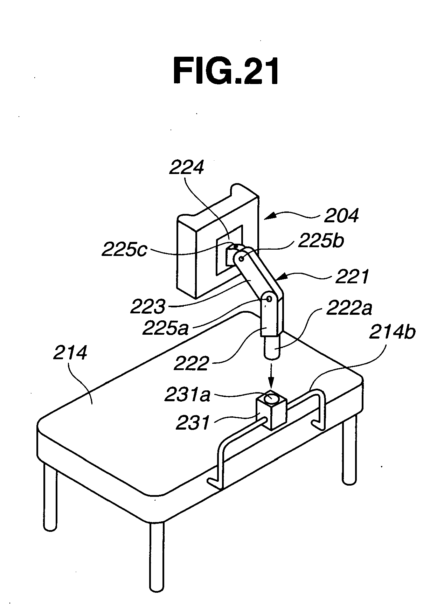

That is, the arm portion 223 of the unit support arm 221 and the unit mounting portion 224 supported at its tip end is rotatably supported by each of the shafts 225a to 225c, and by rotating them, the sense coil unit 204 can be elevated above the bed 214 as shown in Fig. 20, and by moving it in the horizontal direction, the position and direction of the sense coil unit 204 can be set at an optimal position. Also, Fig. 21 shows an example where the unit support arm 221 to which the sense coil unit 204 is mounted is attached to a side guard frame 214b provided at one side of the bed 214 through an arm support block 231 as a receiving member.

The arm support block 231 is fixed to the side guard frame 214b, and on its upper face, a recess portion 231a is formed for supporting the boss shaft 222a projected at the lower end of the unit support arm 21.

In this construction, when an endoscopic inspection or a treatment using an endoscope is to be performed for a patient lying on the bed 214, an operator fixes the side guard frame 214b provided at one side of the bed 214 to one side of the bed 214.

To the recess portion 231a formed on the upper face of the arm support block 231 fixed to the side guard frame 214b, the boss shaft 222a projected at the lower end of the unit support arm 221 is attached. Then, the sense coil unit 204 fixed to the unit support arm 221 is disposed in the vicinity of the patient.

The operator rotates the unit support arm 221 itself in the horizontal direction or rotates in the vertical direction arm portion 223 provided at the unit support arm 221 and the unit mounting portion 224 supported by the its tip end so as to set the sense coil unit 204 at an optimal position.

In the present embodiment, since the arm support block 231 for supporting the unit support arm 221 is fixed to the side guard frame 214b, there is no need to prepare a stand or the like for holding the unit support arm 221.

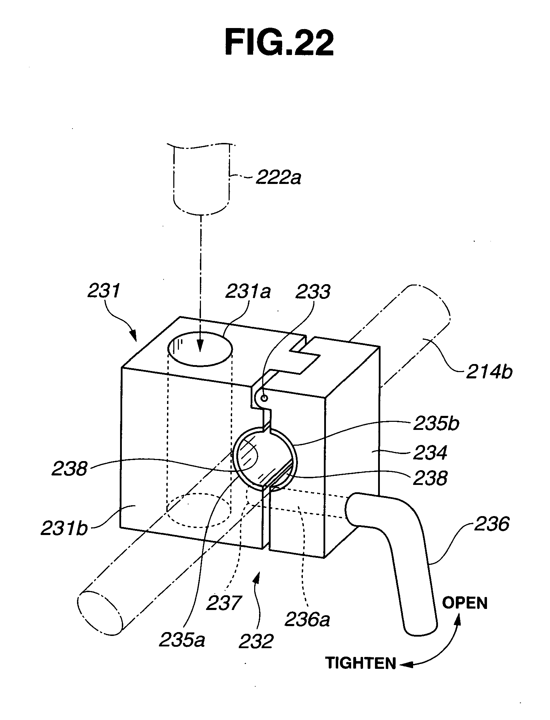

In this case, the arm support block 231 may be configured to be detachably attached to the pipe-state side guard frame 214b. For example, as shown in Fig. 22, a clamp portion 232 is provided at the arm support block 231, and the arm support block 231 is fixed to the side guard frame 214b through the clamp portion 232.

The clamp portion 232 has a clamp member 234 having one end rotatably supported through a hinge pin 233 with respect to a support block portion 231b, and on opposed surfaces of the clamp member 234 and the support block portion 231b, holding recess portions 235a, 235b holding the side guard frame 214b between them are formed. Also, on the inner surfaces of the holding recess portions 235a, 235b, an elastic member 238 such as a rubber sheet is affixed.

Also, a lever 236 is rotatably supported by the clamp member 234. At the tip end of this lever 236, a screw portion 236a is formed and moreover, a female screw portion 237 to be screwed with the screw portion 236a is screwed in the support block portion 231b.

In mounting the arm support block 231 to the side guard frame 214b provided at one side of the inspection bed 214, the operator first rotates the lever 236 in the "open" direction indicated by an arrow in Fig. 22 so as to separate the screw portion 236a formed at the tip end from the female screw portion 237 screwed in the support block portion 231b so that the clamp member 234 is made rotatable around the hinge pin 233.

Then, the operator holds the side guard frame 214b formed in the pipe state between the holding recess portions 235a, 235b formed on the mutually opposed surfaces on the clamp member 234 and the support block portion 231b and rotates the lever 236 in the "tighten" direction indicated by the arrow in Fig. 22.

Then, the screw portion 236a provided at the tip end of the lever 236 is screwed into the female screw portion 237 screwed in the support block portion 231b, and the side guard frame 214b is tightened between the holding recess portions 235a, 235b and clamped. At that time, since the elastic member 238 is affixed to the inner circumference of the holding recess portions 235a, 235b, the side guard frame 214b is not scratched or moreover, not rotated.

After that, the operator inserts the boss shaft 222a projected downward from the stand mounting portion 222 provided at the lower end of the unit support arm 221 into the recess portion 231a drilled in the support block portion 231b so that the unit support arm 221 is supported by the arm support block 231 similarly to Fig. 21.

According to this construction, since the arm support block 231 is made detachable with respect to the side guard frame 214b, the arm support block 231 can be removed when not necessary, which does not lie in the way but improves usability. Also, since the mounting position of the arm support block 231 can be freely determined with respect to the side guard frame 214b, it can be mounted at an optimal position according to the height or the like of the patient.

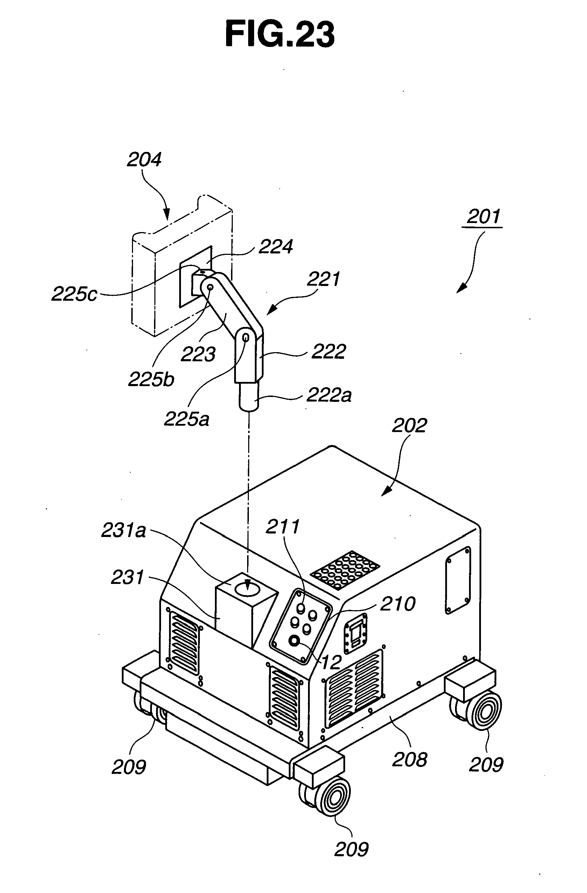

Fig. 23 is a perspective view showing an entire configuration of an endoscope insertion shape detecting device of a fifth embodiment different from the fourth embodiment in Fig. 16. In the above-mentioned fourth embodiment, the device body 202 and the sense coil unit 204 are separated from each other, but in the present embodiment, the arm support block 231 is provided on the front surface of the device body 202, and to this arm support block 231, the boss shaft 222a provided at the lower end of the unit support arm 221 is attached so that the sense coil unit 204 is assembled to the device body 202.

In the present embodiment, the arm support block 231 is provided at the front surface of the device body 202, and only by attaching the boss shaft 222a projected at the lower end of the unit support arm 221 to the recess portion 231a formed at the arm support block 231, the sense coil unit 204 can be easily assembled to the device body 202.

According to this construction, the unit support arm 221 can be used both in the case where it is assembled to the device body 202 and the case where it is used separately from the device body 202, which is convenient in use.

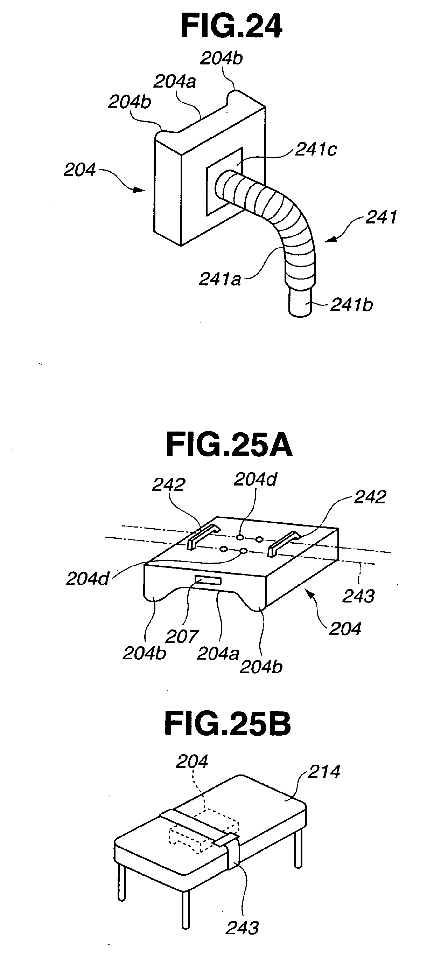

Fig. 24 shows a perspective view of another embodiment of the unit support arm. A unit support arm 241 in the present embodiment has a flexible arm 241 a as holding means, and the boss shaft 222a is provided at the lower end of this flexible arm 241a. Also, a unit mounting portion 241c is provided at the upper end. The boss shaft 222a is, similarly to the fourth embodiment shown in Fig. 16, attached to and supported by the recess portion 231 a formed at the arm support block 231. Also, the sense coil unit 204 is fixed to the unit mounting portion 241 c.

In this construction, the detecting surface 204a of the sense coil unit 204 can be directed to an arbitrary position and direction in a three-dimensional space by bending the flexible arm 241a in the unit support arm 241, which is convenient in use.

Fig. 25A shows another embodiment. The sense coil unit 204 in the present embodiment has a belt passage portion 242 as a fixing member added to the back face of the sense coil unit 204 shown in Fig. 17B.

By additionally forming the belt passage portion 242 on the back face of the sense coil unit 204, in addition to the above-mentioned configuration example shown in Fig. 16, the sense coil unit 204 can be fixed to various portions by using a belt 243 passed through the belt passage portion 242.

Fig. 25B shows an example in which the belt 2043 is wrapped around the inspection bed 14 and the sense coil unit 204 is fixed to the bottom surface side of the inspection bed 14.

According to the present embodiment, only by additionally forming the belt passage portion 42 on the back face of the sense coil unit 204 in the fourth embodiment in Fig. 16, in addition to the use aspect in Fig. 16, the sense coil unit 204 can be fixed using the belt 243 without providing a supporting structure for the sense coil unit 204 at the bed 214.

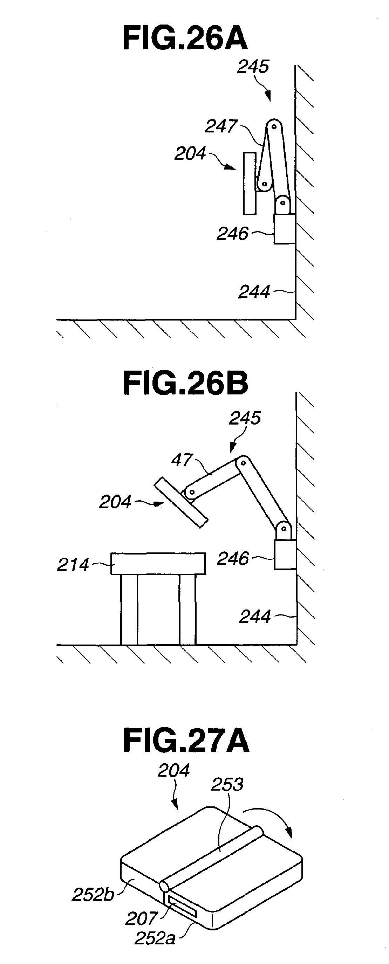

Fig. 26A shows another embodiment. In the present embodiment, a unit support arm 245 which fixes the sense coil unit 204 at the tip end is supported by a base 246 as a receiving member provided at a wall surface 244, for example, provided at the facility.

As a mounting method of the unit support arm 245, similarly to the fourth embodiment, it may be so constructed that a boss shaft is provided at the lower end of the unit support arm 245, a recess portion for supporting the boss shaft is formed at the base 246, and the boss shaft is attached to the recess portion for support.

Since the unit support arm 245 for fixing the sense coil unit 204 is fixed to the wall surface 44 of the facility, when an endoscopic inspection or a treatment using an endoscope is not to be carried out, an arm portion 247 of the unit support arm 245 is folded as shown in Fig. 26A, and the sense coil unit 204 is retreated to the wall surface 244 side so that the facility can be used wide. Also, since there is no need to fix the sense coil unit 204 to the inspection bed 214, the bed 214 can be moved freely.

On the other hand, if a movable bed such as a stretcher is used as the bed 214, if the sense coil unit 204 is fixed to the bed 214, the sense coil unit 204 should be removed every time before bed 214 is to be moved. In this regard, in the present embodiment, since the sense coil unit 204 is fixed to the wall surface 244 side, only the bed 214 can be moved easily.

Moreover, as shown in Fig. 20, when the unit support arm 245 is fixed to the stand 226, since the stand 226 remains in the facility even after the endoscopic inspection is finished, the space in the facility is made narrow by the stand 226, but in the present embodiment, since the sense coil unit 204 is fixed to the wall surface 244 side, the space in the facility can be used wide.

Of course, when an endoscopic inspection or a treatment using an endoscope is to be used, the arm portion 247 is extended by drawing the unit support arm 245, and the sense coil unit 204 can be easily faced on the bed 214, which is convenient in use. In the present embodiment, the base 246 as the receiving member is provided on the wall surface 244 of the facility, but the base 246 may be provided on a floor or a ceiling of the facility. Moreover, the unit support arm 245 may be directly provided on the wall surface, floor surface, ceiling or the like of the facility.

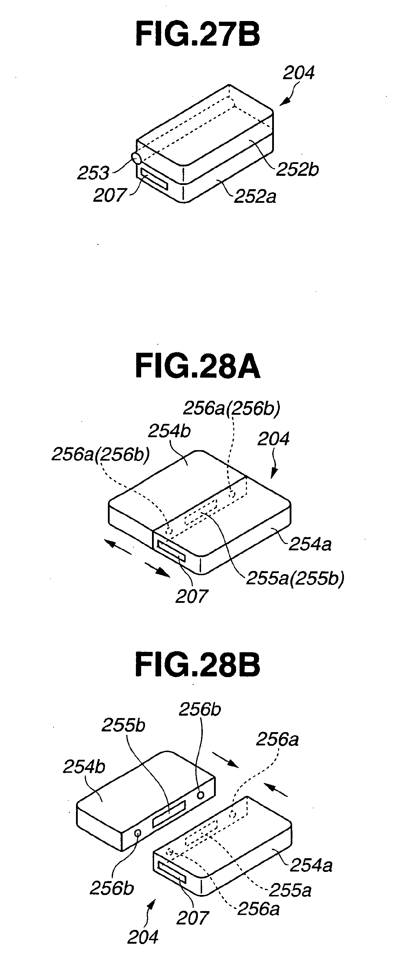

Fig. 27 shows a ninth embodiment of the present invention. The sense coil unit 204 in the present embodiment is configured to be able to be folded in halves at the center.

That is, the sense coil unit 204 in the present embodiment is divided into two unit portions 252a, 252b, and the center portion is connected by a hinge portion 253. The sense coil 205 incorporated in one unit portion 252b (See Fig. 17A or 17B) is connected to the connector receiver 207 provided at the other unit portion 252a through the hinge portion 253.

In the present embodiment, the sense coil unit 204 is divided into the two unit portions 252a, 252b, and the function as the sense coil unit 204 can be exerted by extending the both unit portions 252a, 252b as shown in Fig. 27A, while it can be stored in a compact manner by folding it into halves at the hinge portion 253 as shown in Fig. 27B, when not in use.

Fig. 28A shows a tenth embodiment of the present invention. The sense coil unit 204 in the present embodiment is divided into two unit portions 254a, 254b at the center.

That is, on abutted surfaces of the two unit portions 254a, 254b, connector portions 255a, 255b to be joined to each other are provided, and positioning portions 256a, 256b are provided for male-female fitting to each other. The sense coil 205 incorporated in one unit portion 254b (See Fig. 17A or 17B) is connected to the connector receiver 207 provided at the other unit portion 254a through the connector portions 255a, 255b.

In the present embodiment, when the sense coil unit 204 is to be used, the abutted surfaces of the divided unit portions 254a, 254b are joined to each other as shown in Fig. 28A. Then, the positioning portions 256a, 256b provided on the both abutted surfaces are male-female fitted and positioned, and the connector portions 255a, 255b are connected so as to form the single sense coil unit 204.

On the other hand, when not in use, the unit portions 254a, 254b are separated and the both unit portions 254a, 254b are stacked so as to be stored in a compact manner as shown in Fig. 28.

The present invention is not limited to the above embodiments, but it may be so constructed that the sense coil unit 204 is made as an external unit (coil unit) incorporating a magnetic-field generating element, a magnetic-field detecting element is disposed in an insertion portion of an endoscope, and the insertion shape of the endoscope insertion portion is detected by detecting a magnetic field generated by the magnetic-field generating element by the magnetic-field detecting element on the endoscope insertion portion side.

Embodiments constructed by partially combining each of the above-mentioned embodiments also belong to the present invention.

### Industrial Applicability

When an insertion portion of an endoscope is to be inserted into a body cavity, by arranging a plurality of magnetic-field generating elements inside the insertion portion for position detection so as to calculate and display the shape of the insertion portion, smooth insertion of the insertion portion into the bent body cavity is aided.

## Claims

1. An endoscope shape detecting device comprising:
a transmission block for supplying a driving signal to a magnetic-field generating element arranged at either one of an inside of an endoscope insertion portion and an outside of an endoscope;
a receiving block for receiving a signal obtained by detecting a magnetic field generated by the magnetic-field generating element by a magnetic-field detecting element arranged on the other; and
a control block for carrying out control processing including processing for calculating the shape of the endoscope insertion portion from position information of the magnetic-field generating element or the magnetic-field detecting element arranged in the endoscope insertion portion for a signal from the receiving block, wherein
the receiving block provided with an amplification circuit for at least amplifying a signal detected by the magnetic-field detecting element is formed separately from the transmission block and the control block.

2. The endoscope shape detecting device according to claim 1, wherein
the receiving block is stored in a case different from a case storing the control block and the control block so that the receiving block is formed separately from the control block and the control block.

3. The endoscope shape detecting device according to claim 1, wherein
the receiving block has the amplification circuit for amplifying a signal detected by the magnetic-field detecting element and an analog/digital conversion circuit for converting an analog signal amplified by the amplification circuit to a digital signal.

4. The endoscope shape detecting device according to claim 3, wherein
the receiving block further has a serial signal conversion circuit for converting the digital signal to a serial signal.

5. The endoscope shape detecting device according to claim 1, wherein
the receiving block is connected to a control device incorporating the transmission block and the control block through a signal transmission cable for transmitting the signal at least amplified by the amplification circuit.

6. The endoscope device according to claim 3, wherein
the receiving block transmits a digital signal converted by the analog/digital conversion circuit to the control block.

7. The endoscope device according to claim 4, wherein
the receiving block transmits the serial signal to the control block.

8. The endoscope device according to claim 3, wherein
the control block transmits a control signal to the analog/digital conversion circuit of the receiving block.

9. The endoscope device according to claim 3, wherein
the control block has a serial/parallel conversion circuit for converting the serial signal to a parallel signal.

10. The endoscope shape detecting device according to claim 1, wherein
the receiving block has an optical signal conversion circuit for converting the signal to an optical signal.

11. The endoscope shape detecting device according to claim 3, wherein
the receiving block has an optical signal conversion circuit for converting the digital signal to an optical signal.

12. The endoscope shape detecting device according to claim 10, wherein
the receiving block carries out signal transmission by an optical signal to the control block.

13. The endoscope shape detecting device according to claim 1, wherein
the receiving block has a wireless circuit for transmitting the signal in a wireless manner.

14. The endoscope shape detecting device according to claim 3, wherein
the receiving block has a wireless circuit for transmitting the signal in a wireless manner.

15. The endoscope shape detecting device according to claim 13, wherein
the receiving block transmits the signal to the control block in a wireless manner.

16. The endoscope shape detecting device according to claim 1, wherein
the receiving block is stored in a case separate from a detecting unit storing the plurality of magnetic-field detecting elements and is detachably connected to the detecting unit through a signal cable.

17. The endoscope shape detecting device according to claim 1, wherein
the receiving block is stored in a shield case having a function of electromagnetically shielding at least at the amplification circuit portion.

18. The endoscope shape detecting device according to claim 1, wherein
the transmission block is stored in a shield case having a function of electromagnetic shielding.

19. The endoscope shape detecting device according to claim 1, wherein
in the receiving block, mounting means which can be detachably attached to a patient to be inspected by the endoscope is provided.

20. The endoscope shape detecting device according to claim 1, wherein
the receiving block is provided at a bed on which a patient to be inspected by the endoscope is lying.
